# EUROPEAN PATENT SPECIFICATION

(11) **EP 1 397 168 B1**
(45) Date of publication and mention of the grant of the patent: **25.05.2011**
(21) Application number: 02736364.7
(22) Date of filing: 29.05.2002
(51) Int. Cl.: A61L 27/06, A61L 27/30

(54) **MODIFIED TITANIUM OXIDE SURFACE OF IMPLANTS**
TITANOXID HALTIGES IMPLANTAT
IMPLANT DE TITANE AYANT EN SURFACE UN OXIDE DE TITANE MODIFIE

(30) Priority: 29.05.2001 SE 0101910; 12.12.2001 SE 0104213; 12.12.2001 US 339310 P
(43) Date of publication of application: 17.03.2004
(73) Proprietor: Elos Medtech Pinol A/S, 3330 Gørløse (DK)
(72) Inventor: SUL, Young-Taeg, S-436 39 Askim (SE)
(74) Representative: Hansen, Anders Sandgaard
(86) International application number: PCT/SE2002/001024
(87) International publication number: WO 2002/096475

(56) References cited:
- WO-A1-00/72777
- YOUNG-TAEG SUL ET AL.: 'Qualitative and quantitative observations of bone tissue reactions to anodised implants' BIOMATERIALS vol. 23, 2002, pages 1809 - 1817, XP004341884
- YOUNG-TAEG SUL ET AL.: 'Characteristics of the surface oxides on turned and electrochemically oxidized pure titanium implants up to dielectric breakdown: the oxide thickness, nicropore configurations, surface roughness, crystal structure and chemical composition' BIOMATERIALS vol. 23, 2002, pages 491 - 501, XP004348056
- YOUNG-TAEG SUL ET AL.: 'The electrochemical oxide growth behaviour on titanium in acid and alkaline electrolytes' MEDICAL ENGINEERING & PHYSICS vol. 23, 2001, pages 329 - 346, XP002970661
- SUL Y.-T. ET AL.: 'Oxidized implants and their influence on the bone response' JOURNAL OF MATERIALS SCIENCE: MATERIALS IN MEDICINE vol. 12, 2001, pages 1025 - 1031, XP002970674
- DATABASE CAPLUS [Online] DOC. NO. 117:55890 KATO MAKOTO ET AL.: 'On the dental application of titanium-base alloy. 6. Effect of the composition of electrolytic solutions in anodizing the material', XP002970643 Retrieved from STN Database accession no. 1992:455890 & NAGOYA KOGY GIJUTSU SHIKENSHO HOKOKU vol. 40, no. 8/9, 1992, pages 282 - 290
- MILENA FINI ET AL.: 'In vitro and in vivo behaviour of Ca- and P-enriched anodized titanium' BIOMATERIALS vol. 20, 1999, pages 1587 - 1594, XP002970662

## Description

### Field of the invention

The present invention relates to calcium-containing, phosphor-containing and/or sulphur-containing osteoconductive/ osteoinductive titanium/titanium alloy implants based on surface oxide chemistry modifications of bone-anchored titanium/titanium alloy implants. The invention also relates to a process of producing said implants.

### Background of the invention

Titanium metal is so far the most successfully osseointegrated implant material in long-term clinical performance [Adell R. et al., 1990]. Despite excellent biocompatibility of thin native oxide films on titanium implants [Kasemo B. et al., 1986 and Johansson C. B. 1991], however, it is generally known that native titanium oxide seldom forms a direct chemical bond to bone tissue (often defined as inert ceramic biomaterial) [Li J. et al., 1998]. Various surface processing technologies such as plasma spraying, simple immersion/soaking method, ion implantation beam associated deposition have been applied to improve biocompatibility of titanium implants. Developments of clinical implants based on alterations of the surface chemical properties have been associated with so called 'bioactive implant' coated with bioactive materials such as various species of hydroxyapatite, calcium phosphate compound, bioglass and bioceramic on metal implants [Hench et al., 1990]. However, a number of studies have reported poor long-term biological stability, for instance, a delamination (intra- and interfacial fracture) of such coating materials as well as biodegradation [Albrektsson T., 1998 and Gottlander M., 1994]. Among many surface modification methods, anodic oxidation is a valuable process to enforce the multifactorial biocompatibility such as the oxide thickness, chemical composition, surface morphology, crystallinity, surface roughness change and dielectric constant of the surface oxide in titanium implants [Sul et al. 2000a and 2001b]. The average maximum stress recorded 33 MPa in the mechanical properties of a thick oxide layer [Hala J et al 2000]. Michiaki et al. (1989) and Seishiro (1989) reported the thicker oxide layer in few micrometer to few tens of micrometer with few µm of pore size fabricated in the H₂SO₄ + H₃PO₄ mixed electrolyte system. The cross-cut structures of the thicker oxide layer measured with Scanning Electron Microscopy (SEM) was characterized to have the network structure of pore channels and connected channel branches.

U.S. 5,478,237 discloses an anodic oxide film containing both calcium and phosphor, which by a further hydrothermal treatment provides a film comprising a calcium phosphate compound such as hydroxyapatite.

WO 98/51231 discloses modified titanium oxide layers of about 10-200 µm and an increased surface oxide crystallinity and roughness.

U.S. 5,354,390 discloses a process of forming an oxide layer using anodic oxidation followed by a heat treatment.

WO 00/72775 discloses coatings including calcium phosphate compounds. According to WO 00/72777 and WO 00/72776, the surface of oxide layer on the titanium includes about 20 % titanium, about 55 % oxygen and 20 % carbon, the rest of the layer is composed of titanium oxide. The used electrolytes are inorganic and organic acid, for instance the H₂SO₄ + H₃PO₄ mixed electrolyte system. Cross-sections of the titanium oxide layer disclosed in these documents show a very thick layer exhibiting a network of channels, extension of the channels and connected channel branches. However, the purpose of the channels is mainly to allow the administration of a bone-stimulating agent.

There exists a need for a titanium/titanium alloy implant having an oxide layer exhibiting improved surface properties for a faster and stronger osseointegration.

### Summary of the invention

The object of the present invention is to produce osteoconductive/osteoinductive oxide properties of titanium/titanium alloy implants, leading to faster and stronger osseointegration in clinical performance of bone-anchored titanium/titanium alloy implants and improved clinical success for long-term functional loading in the human body.

This object has been solved by providing a osteoconductive/osteoinductive titanium/titanium alloy implant comprising an additional element in the titanium oxide, obtained by anodic oxidation, which is characterised in that said additional element being a single one element chosen from the group consisting of calcium, phosphor or sulphur and that said implant exhibits a cross-section of the osteoconductive/ osteoinductive oxide layer, which consists of a double layer structure of an upper porous layer with anodic incorporation of the additional element and a lower compact barrier layer of the titanium oxide with anodic incorporation of the additional element,
wherein the upper porous layer exhibits more than about 11 % porosity and less than about 30 % porosity, and whereby the thickness of said osteoconductive/osteoinductive double layer-structured oxide containing an additional element is from 300 to 3000 nm, wherein said upper layer has a thickness of less than 1000 nm, and said lower barrier layer has a thickness ranging between 300 to 2000 nm.

In a preferred embodiment thereof the upper layer has a thickness of 100-500 nm.

In a preferred embodiment thereof the lower barrier layer has a thickness ranging between 600-1500 nm.

In a preferred embodiment thereof the thickness of said osteoconductive/osteoinductive double layer-structured oxide containing an additional element is between 800 and 1500 nm.

In a preferred embodiment thereof the lower barrier layer does not include any pores/craters or channels.

In a preferred embodiment thereof the upper porous layer exhibits about 15 % porosity

In a preferred embodiment thereof the crystal structure of titanium oxide is amorphous and/or amorphous and anatase and/or amorphous, anatase and rutile.

In a preferred embodiment thereof the relative concentration of the additional element incorporated into the anodic oxide layer formed on titanium/titanium alloy implants increases with the thickness of the oxide layer containing an additional element, wherein the relative concentration of the additional clement of the oxide layer of said implant is between 1 % and 50 %.

In a preferred embodiment thereof the relative concentration of the additional element in the oxide layer of said implant is between 1 % and 25 %.

A further aspect of the invention relates to a process of producing osteoconductive/osteoinductive titanium/titanium alloy implants comprising an additional compound as a titanium oxide component, wherein the additional compound is chosen from the group consisting of calcium, phosphor or sulphur, using a electrochemical oxidation method, micro arcing oxidation, comprising the steps of:
a) controlling the anodic forming voltage transient with slope (*dV*/*dt*)*,* oxide growth constant (α, nm/V), the current efficiency (nm.cm²/C), the anodic oxide forming rate (nm/sec);
b) controlling the intensity and extent of breakdown phenomenon and breakdown voltage;
c) controlling the incorporation of either calcium, phosphorous or sulphur into [the] a double layer structure of a upper porous and a lower barrier oxide layer by colloidal deposition mechanism,
d) controlling reinforcement of the mechanical properties of said calcium-containing oxide for long term functional loading in the human body - to strictly confine the anodising time from the first onset of micro arcing phenomenon on the titanium/titanium alloy anode to near anodic forming voltage transient with slope *dV*/*dt* ≈ 0.3, during the different phases of the anodic process;
e) detecting the first onset of said micro arcing phenomenon of said process during the oxidation;
f) maintaining said process by near anodic forming voltage transient [with the slope,] *dV*/*dt* being about 0.3, as to produce an oxide layer on said titanium/titanium alloy implant exhibiting a double layer structure of an upper porous layer and a lower compact barrier layer,
g) and interrupting the oxidation process when dV/dt is between 0.2-0.4.

In a preferred embodiment thereof current density (mA/cm²) ranges from 30 to 4000 mA/cm², preferably from 60 to 2000 mA/cm².

In a preferred embodiment of the process the anodic forming voltage ranges from 30 to 500 V, preferably from 250 to 500 V.

In a preferred embodiment the process the temperature during the process ranges from 10 to 100 °C, preferably from 10 to 50°C.

In a preferred embodiment of the process the temperature during the process ranges from 10 to 50 °C.

In a preferred embodiment of the process for producing a osteoconductive/osteoinductive titanium/titanium alloy implant comprising calcium as a titanium oxide component, wherein said titanium /titanium alloy implant is electrochemically anodically oxidised in (a) at least one calcium-containing electrolyte and (b) ethylene diamine tetra acetic acid to yield a calcium-containing implant, whereby preferably the calcium-containing electrolyte used in the anodising process is chosen from the group consisting of: calcium acetate, calcium carbonate, calcium chloride, calcium citrate, calcium glycerophosphate, calcium hydroxide, calcium lactate, calcium nitrate, and calcium sulphate.

In a preferred embodiment of the later process stages the anodic oxidation is performed at an alkaline pH value.

In a preferred embodiment of the later process stages the mixture used in the anodic oxidation process also includes weak acid electrolytes, chosen from the group consisting of boric acid, maleic acid, malonic acid, oxalic acid and succinic acid.

The porous upper layer exhibits an open structure comprising a plurality of shallow craters.

The oxides produced by the process according to the invention demonstrate excellent mechanical strength and do not show a delamination of the oxide film and oxide particles internalised by the inflammatory cells such as microphage and multinuclear giant cell.

The implants in the present invention takes advantage of the stronger and faster osseointegration as proved in animal studies by strong bonding reactions between bone and the modified surface chemistry of titanium oxide by using process according to the invention, and consequently promote highly successful modality in clinical performance of osseointergrated titanium implants. In addition, the implant surfaces in the present invention have the reinforced mechanical strength and overcome a drawback such as the biological delamiantion/ resorption of the coated calcium phosphate and hydroxyapatite on implants, resulting in clinical loosening of implants in bole.

### Brief description of figures

The present invention will now be further illustrated by means of drawings enclosed herewith, wherein:
- **Fig. 1**.: shows a schematic drawing of the equipment setup.
- **Fig. 2.**: shows the anode voltage increase in time. The line shows three distinct zones with different slope: Zone 1 is linear with the highest slope; zone 2 is non-linear with slope *dV*/*dt* ≥ 0.3; and zone 3 is non-linear with d*V*/d*t* < 0.3. Oxide growth characteristics in the three electrochemical zones are exemplified by Scanning Electron Micrographs (SEM) of calcium-incorporated layers. a = barrier oxide film produced in zone 1; b and c = the optimal surface prepared at d*V*/d*t* ≥ 0.3 of zone 2; e = crack-propagated surface obtained in zone 3.
- **Fig. 3.**: shows the electrochemical oxide growth in zone 2 only, and its dependence on various parameters as described in the text (numbers 1-12). The incidence of slope d*V*/d*t* = 0.3 is indicated in each plot, as is the electric current density.
- **Fig. 4.**: shows Transmission Electron Micrographs (TEM) of ultramicrotomed cross-sections of the three oxide layer types, in order to demonstrate the double layer structure of each consisting of the upper porous and lower barrier layer, respectively. U = upper porous layer with pores/craters, L = lower barrier layer with no pores/craters, I = titanium/oxide interface, Ti = titanium bulk of the implant, D = width of the pore and H = height of the pore. Thickness of L»U and size D»H.
- **Fig. 5.**: shows SEM pictures of the surface of Ca, P, and S implants, respectively.
- **Fig. 6.**: shows Light microscopic comparison between an implant produced by conventional anodic oxidation (fig. 6a) and a calcium-containing implant according to the present invention (fig. 6b). In 6a internalised oxide particles (arrows) are clearly visible in macrophages but no oxide particles are present in 6b. The implants by this invention also exhibit superior osteoconductivity (larger area of bone to implant contact). Bar = 100 µm, Ip=implant, B = bone
- **Fig. 7.**: shows High resolution X-ray Photoelectron Spectroscopy (XPS) at the Ca 2p_{1/2}, P 2p_{3/2}, and S 2p peak positions of the Ca, P, and S implants, respectively.
- **Fig. 8.**: shows Auger Electron Spectroscopic (AES) measurements of Ca, P, and S concentration depth profiles of the Ca, P and S implants, respectively. U = upper porous layer with pores/craters, L = lower barrier layer with no pores/craters.
- **Fig. 9.**: shows X-ray Diffraction (XRD) angle measurements of the implants by this invention and control implants, respectively.
- **Fig. 10.**: shows mean values of peak removal torque measurements of the implants according to this invention compared to control implants. * indicates statistically significant differences (p < 0.05), and ** indicates highly statistically significant differences. (Ca implants) (p = 0.0001). Standard errors of the mean are given as bars.
- **Fig. 11.**: shows mean values (%) of bone to metal contact (BMC) measurements of the implants according to this invention compared to control implants. ** indicates highly statistically significant differences (p < 0.001). Standard errors of the mean (SEM) are given as bars.
- **Fig. 12**.: shows light microscopic observations showing the much higher osteoconductive/ osteoinductive properties of the present implants (1, 4, and 6) when compared to control implants (2, 3, and 5).

### Detailed description of the invention

### The micro arc anodic oxidation (MAO) process

The present invention produces osteoinductive/osteoconductive titanium/titanium alloy implants with additional components (additives: simple calcium, phosphorus, or sulphur compounds) electrochemically incorporated into the titanium oxide surface layer.

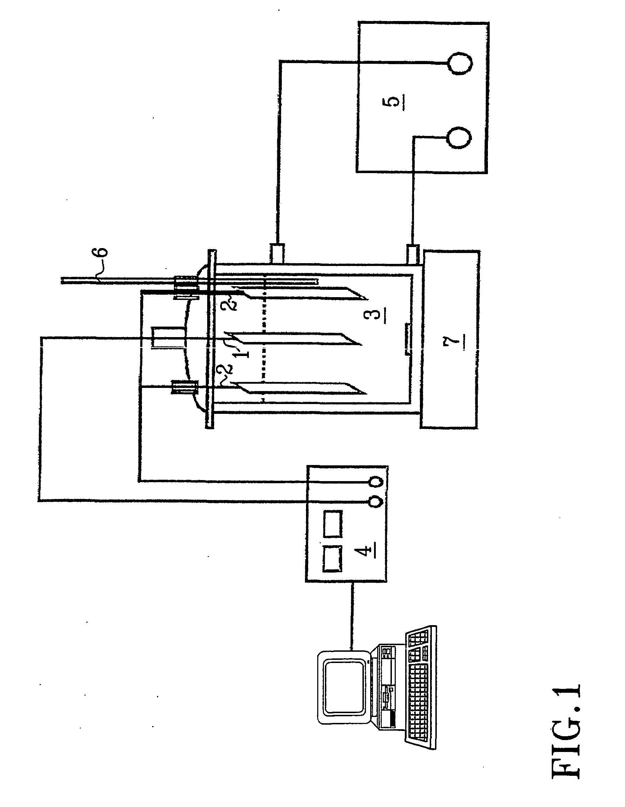

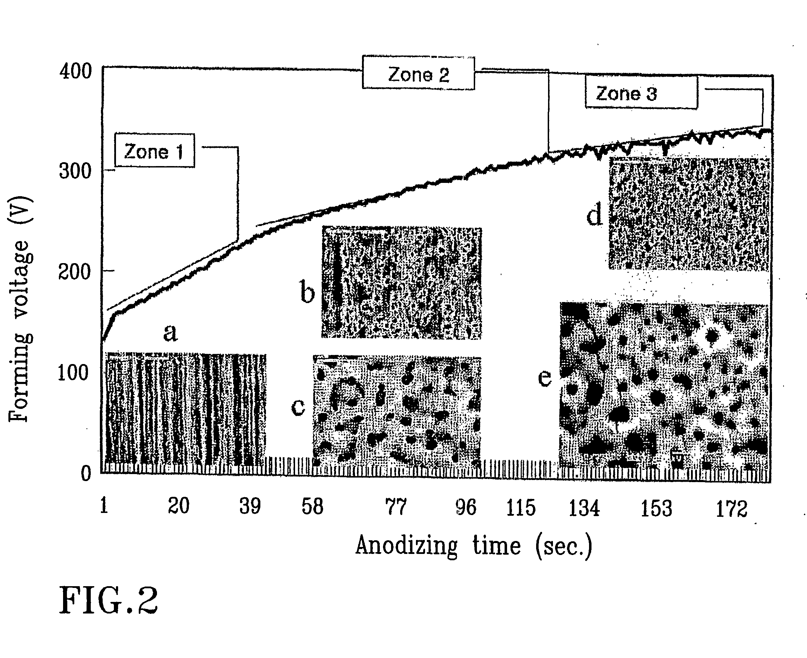

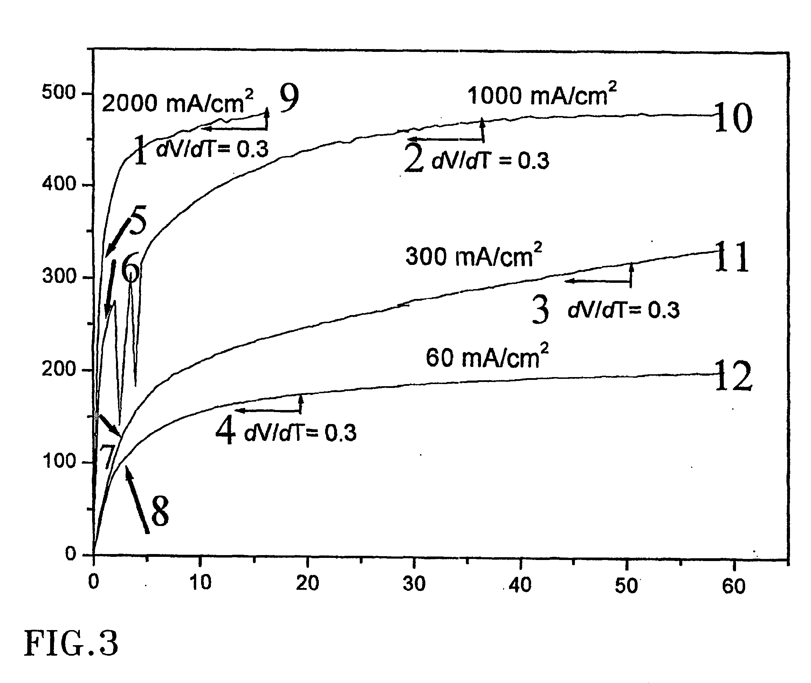

As shown in Fig. 1, the MAO process according to the present invention was performed using an equipment provided with a titanium anode 1 and a platine cathode 2 in an electrolyte 3. A DC power supply 4 was connected to a computer in which electrical current and voltage was recorded every 0.5 second. The temperature was measured using a thermometer 6 and the desired temperature was maintained using a cooling system 5. The electrolyte was stirred using a magnetic stirrer bar 7 on a magnetic stirrer plate 8. During the process the anodic oxide-forming voltage increases with time during three distinguishable phases ("zones") with different slopes d*V*/d*t* (Fig. 2): In zone 1 the voltage increases linearly; zones 2 and 3 display transient slopes of dV/dt ≥ 0.3 and d*V*/d*t* ≥ 0.3, respectively. It was found (see below) that the best results were obtained by strictly confining the anodising time from the first onset of the MAO phenomenon to near the boundary between zones 2 and 3 in Fig. 2, where the slope of the anodic voltage transient is approximately 0.3. This was orchestrated by controlling the following parameters (Fig. 3), given in order of impact on the process: electrical current density > electrolyte concentration > surface area ratio of anode to cathode > electrolyte temperature ≥ agitation speed > chemical composition of the titanium/titanium alloy electrode (ASTM grade 1- 4).

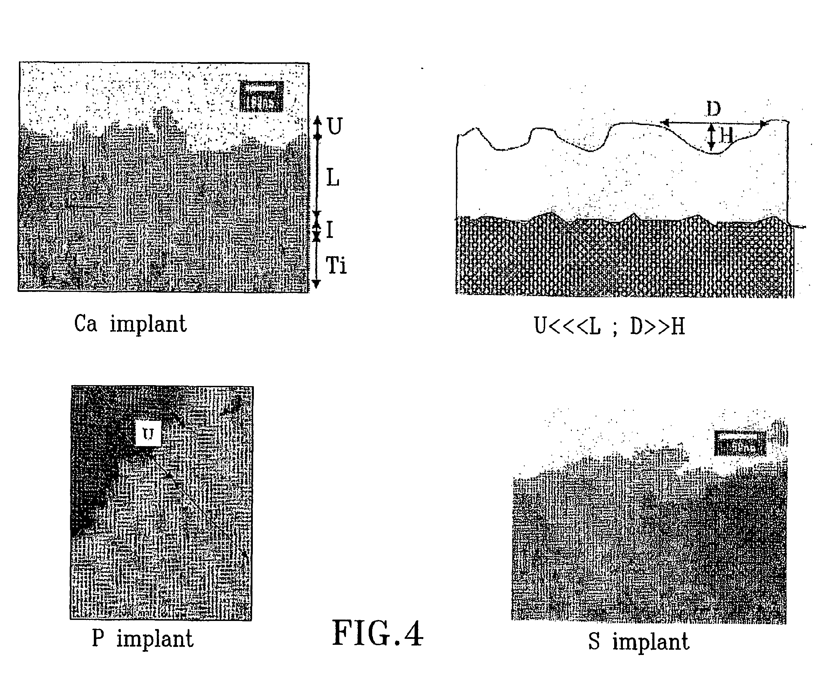

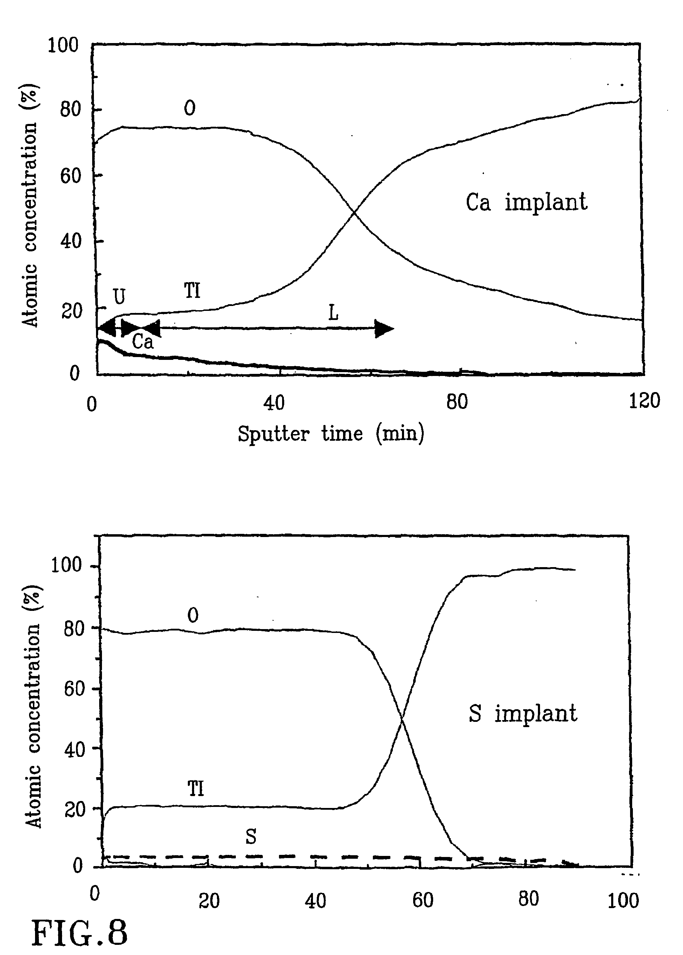

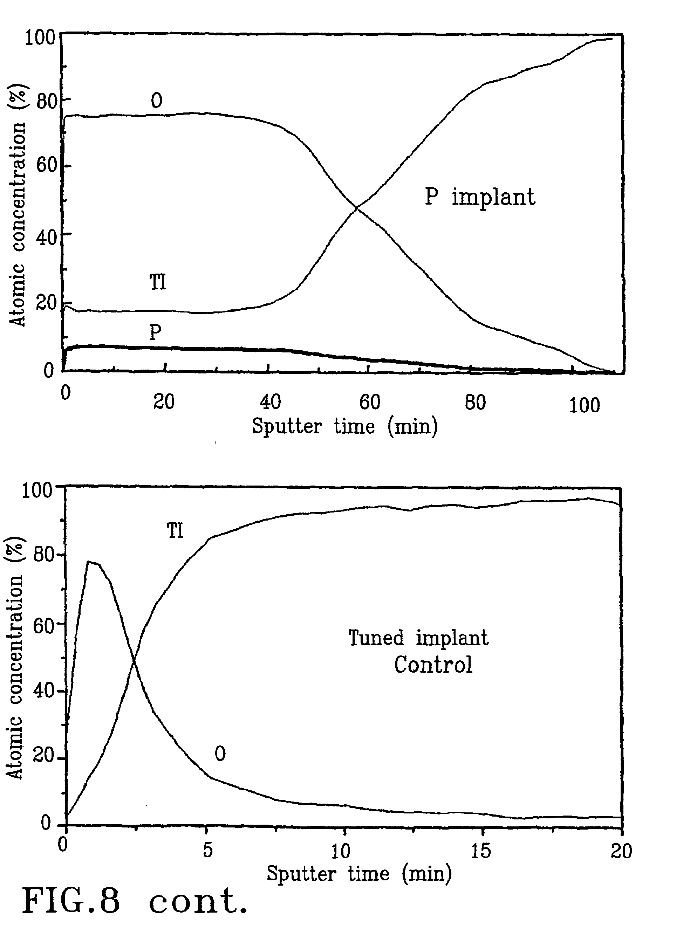

In zone 1 (Fig. 2), the oxide layer grows linearly with the anodic voltage according to the sample relationship *D* = α*V*, where *D* is the thickness, α is a growth constant, and *V* is the voltage. The oxide layer obtained contains lower concentrations of additives (L in the A. depth profiles of Fig. 8) than the layers obtained in zones 2 and 3, and the morphological structure is that of a dense nonporous barrier devoid of pores, craters, and channel networks. This is clearly seen in SEM and TEM micrographs (a in Fig. 2 and L in Fig. 4, respectively). The thickness of this layer is usually between 200-1000 nm (Figs. 4 and 8).

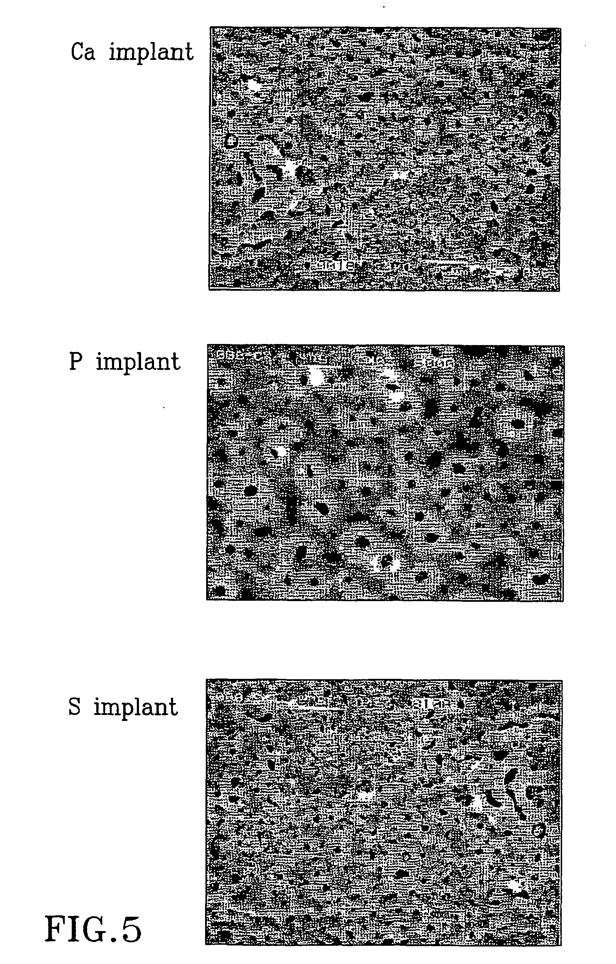

Continuing the anodic oxidation into zone 2 produces an additional oxide layer with different properties on top of the innermost barrier layer. This outer layer has a multitude of pores and craters (b and c in Fig. 2, Fig. 5, and U in Fig. 4) and a higher concentration of additives (U in Fig. 8). Furthermore, it is thinner in than the innermost layer with a thickness lower than 100 nm (Figs. 4 and 8), and on all MAO-treated surfaces studied the outer layer constituted less than 40% of the two layers taken together.

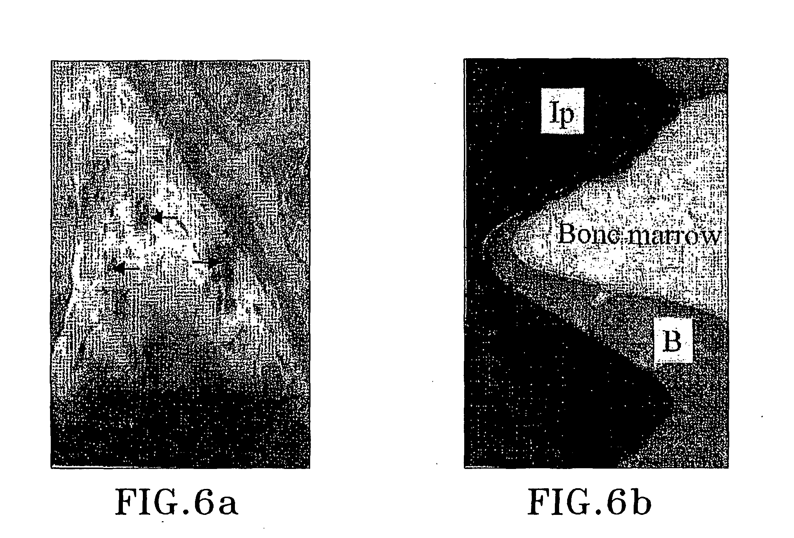

Stopping the electrochemical oxidation process at d*V*/d*t* ≈ 0.3, encompassing zones 1 and 2, thus yields a double-layered oxide surface with optimal morphological and mechanical properties, and this is the case for all three types of additives. If, however, the anodic oxidation is allowed proceed into zone 3 (d*V*/d*t* < 0.3) the morphology of the oxide layer obtained is somewhat similar to that of zone 2 but it is thicker, and has larger and more numerous pores, craters, and channels (d in Fig. 2). This is the result of the extended breakdown of the zone 3 anodic oxide layer. Such a structure clearly has inferior mechanical properties than those of zones 1 and 2 as is demonstrated by the microcracks in Fig. 2. In addition, it was detected in animal experiments that such a thick oxide film easily delaminates, and the oxide particles released may be internalised into inflammatory cells such as macrophages and multinuclear giant cells (Fig. 6a). On the other hand, the oxide layers with Ca, P, and S additives produced by the MAO process in the present patent claims do not leave unwanted oxide particles in the tissue (Fig. 6b) and they demonstrate excellent mechanical strength, as presented in the experimental section (Table 2).

### Electrolytes

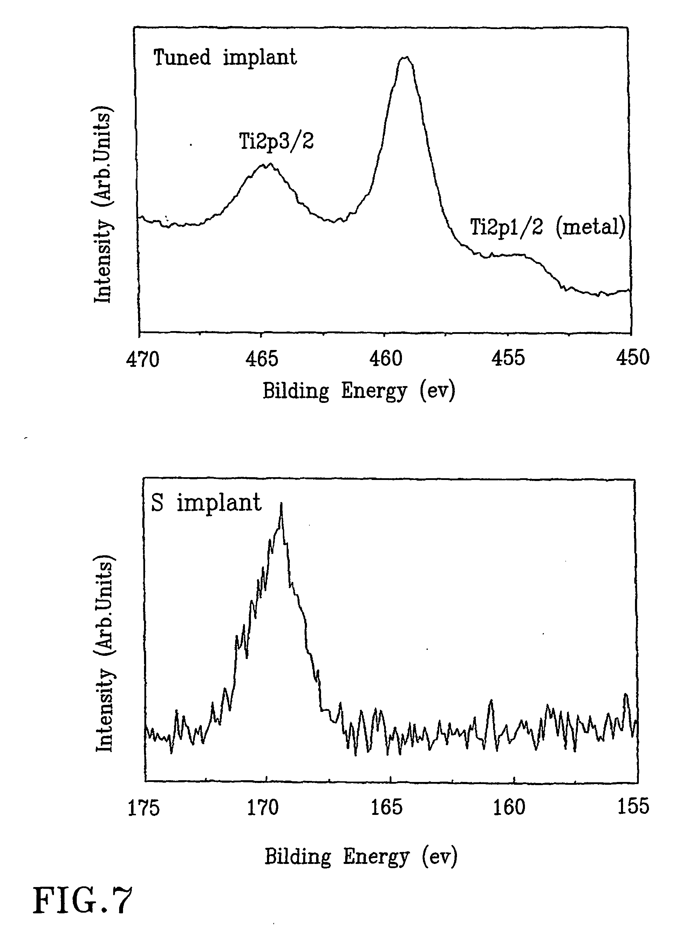

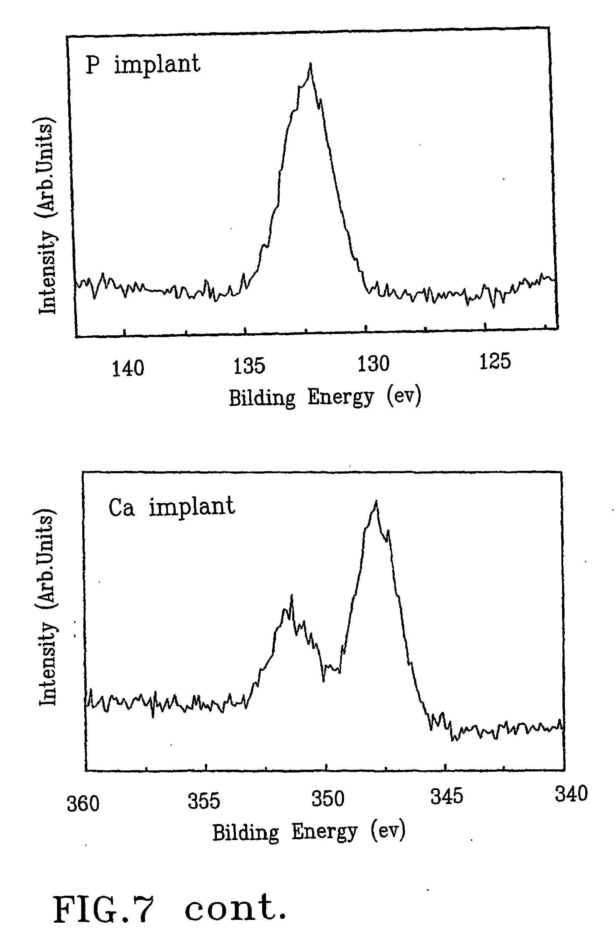

The chemical nature of the electrolyte in the electrochemical cell is of less importance, meaning that a large number of Ca, P, and S-containing salts can be used to equally good results as exemplified for calcium in the Experimental section (Table 1). Single electrolytes are used in the solution but any mixture of two or more relevant compounds may also be used. Specifically for calcium, which, as opposed to the phosphorus and sulphur compounds, incorporates into the oxide layer as a one-atomic positively charged ion (Ca²⁺), simple electrolytes (examples 1-5 in Table 1) gave a relative amount of calcium incorporation of less than 10 %, as detected by XPS and AES (Figs. 7 and 8, respectively). For the purpose of increasing the calcium concentration in the oxide layer, the chelating agent EDTA (ethylene diamine tetraacetic acid) was added to the electrolyte solution and the pH adjusted to between 7 and 12 (examples 7-10 in Table 1). Use of EDTA in this system requires an alkaline pH to give good mechanical properties of the oxide layer.

### Calcium (Ca) implants

The Ca implants produced by the process according to the present invention were characterised with various surface analysis techniques.

The chemical composition of the Ca implant as measured by XPS clearly reveals that calcium is a main added constituent of the surface (Ca 2p in Fig. 7). The spectrum shows a doublet peak from the Ca orbitals 2p_{1/2} and 2p_{3/2} at 351.4 eV and 347.8 eV, respectively (Ca implants) The present Ca peak positions may indicate that Ca is present in calcium titanates such as CaTiO₃. Also, as shown in Fig. 8, AES measurements clearly demonstrate the concentration depth distribution of Ca throughout the oxide layer, which proves that calcium/calcium compounds incorporate into the titanium oxide during said process (maybe by a mechanism of colloidal deposition). The relative atomic concentration of Ca is in the range of 0.5 to 48 %.

The outer porous layer exhibits between 11 % and 30 % porosity, preferably about 15 %. In general, porosity is defined a total area of the opening pores /a total of the scanned area ×x 100 in %. However, in this invention, the porosity was defined as the total area of the opening pores /the total scanned area (3 × 20 µm × 26 µm) in %.

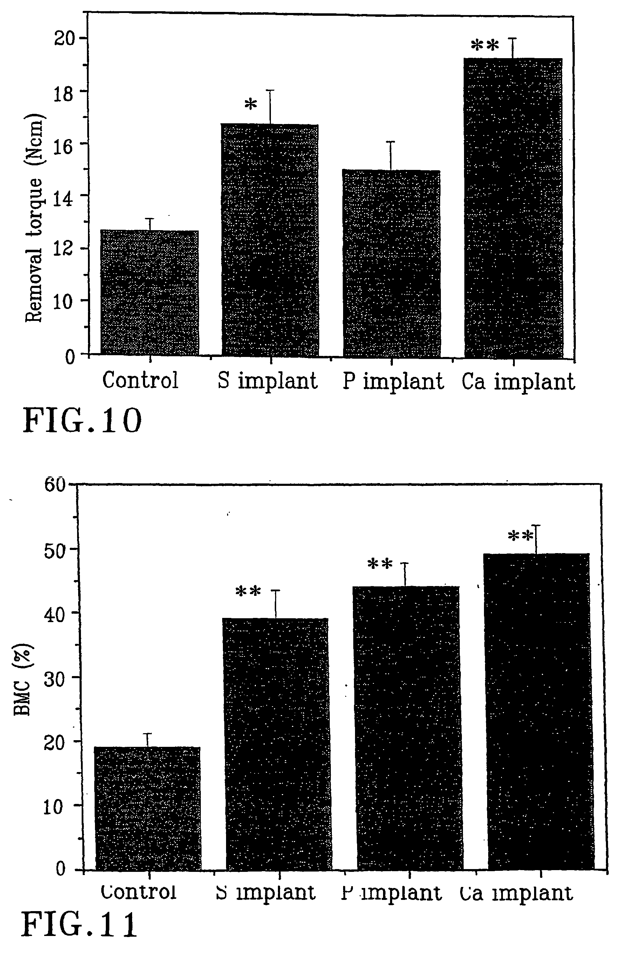

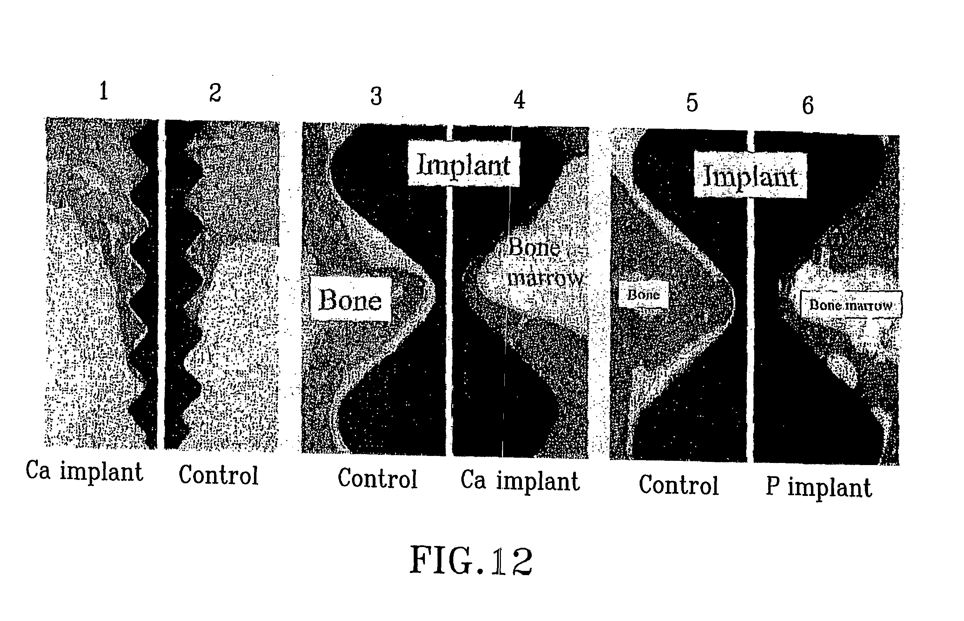

Osseointegration proved considerably faster and stronger for the implants according to this invention than for conventional machine-turned implants (Figs. 10-12). Ca implants in particular demonstrated the strongest integration with bone tissue: a 240 % increase of removal torque (Fig. 10). Furthermore, Ca implants also demonstrated excellent osteoconductivity; the highest degree of mineralization and the closest direct bone to implant contact (Fig. 11), with a 273 % (mean value) increase of bone to metal contact (BMC) in all implant threads (Fig. 12), and a 145 % (mean value) increase in the ratio of inside to outside bone area (Inside/Outside; %) after 6 weeks of implant insertion in rabbit model (see the experimental examples below).

### Phosphorus (P) implants

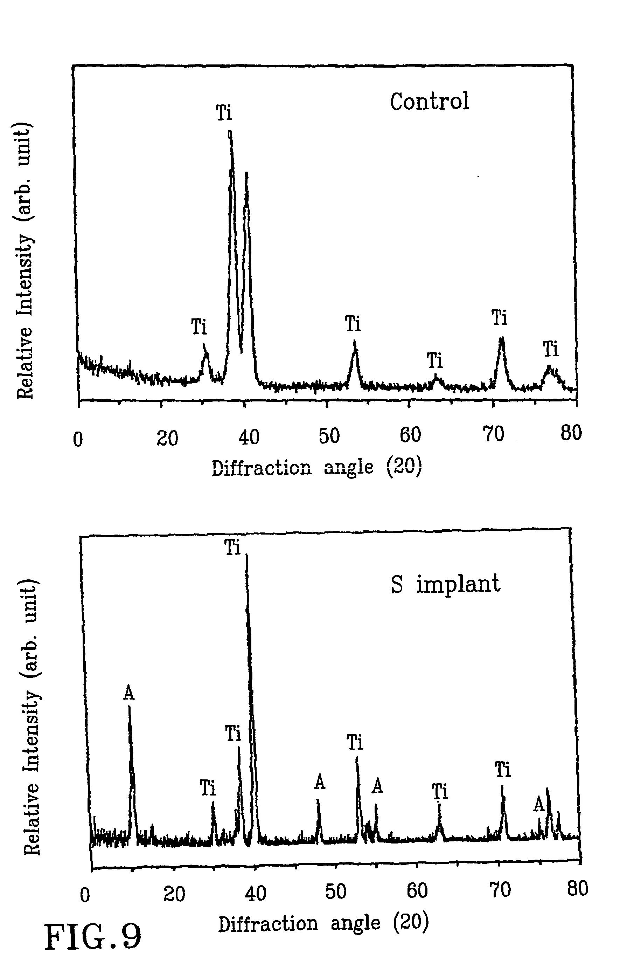

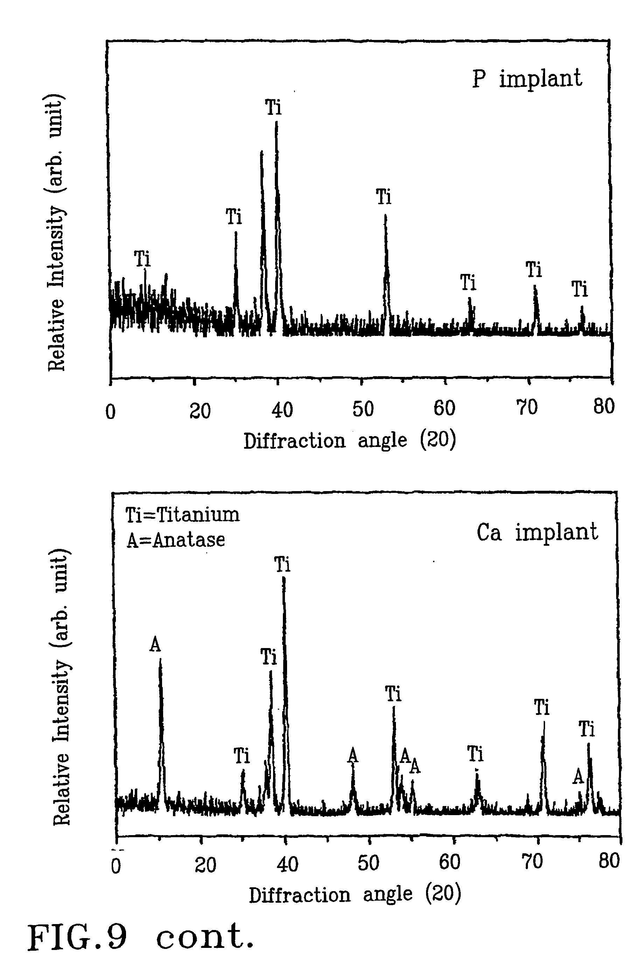

The MAO process according to the present invention can also be used for producing phosphorus-containing implants (XPS surface analyses in Fig. 7). The outer porous layer of the dual-layered oxide surface (U in Fig 4, and Fig. 5) has a thickness below about 1000 nm, preferably 100-500 nm. The lower barrier layer (L in Fig 4) has a thickness between about 300 nm and 2000 nm, preferably 600-1500 nm. The total thickness of both layers typically ranges from 300 to 3000 nm but should preferably be between 600 and 1500 nm (Ti and O in AES depth profile of Fig 8). The outer porous layer exhibits between 11 % and 30 % porosity, preferably about 15 %. The relative phosphorus concentration of the oxide layer of said implant is between 1 % and 30 %, preferably between 3 % and 15 % (P in AES depth profile of Fig. 8). The relative phosphorus concentration/amounts incorporated into the anodic oxide layer increases with layer thickness. The crystal structure is amorphous, and/or amorphous and anatase, and/or amorphous, anatase, and rutile (XRD analysis in Fig. 9).

Phosphorus-containing electrolytes used in the anodising process, singly or in any mixture, are chosen from the group consisting of: phosphoric acid, glycerophosphate, sodium phosphate, sodium pyrophosphate, sodium phosphinate, ammonium phosphate, and potassium phosphate.

### Sulphur (S) implants

In complete analogy with the above, the MAO process according to the present invention can be used for producing implants with sulphur-containing compounds incorporated into the titanium oxide surface. The porous outer layer (U in Fig. 4, and Fig. 5) has a thickness below 1000 nm and should preferably be 100-500 nm, with a porosity between 11-30 %, preferably about 15 %. The lower barrier layer (L in Fig. 4) has a thickness between 300-2000 nm, preferably 600-1500 nm. Total thickness of both layers typically ranges from 300 to 3000 nm, preferably from 600 and 1500 nm (S in Fig. 8). Relative sulphur concentration of the oxide layer is between 1 % and 30 %, preferably between 3 % and 15 % (Fig. 8). The relative sulphur concentration/amounts incorporated into the anodic oxide layer increases with layer thickness. The crystal structure is amorphous, and/or amorphous and anatase, and/or amorphous, anatase, and rutile (Fig. 9).

Sulphur-containing electrolytes used in the anodising process, singly or in any mixture, are chosen from the group consisting of: sulphuric acid, potassium sulphate, sodium sulphate, sodium thiosulphate, sodium hydrosulphite, and sodium pyrosulphite.

The present invention will now be further illustrated by means of the following non-limited examples.

### Experimental section

Before the MAO process, all samples were degreased with trichlorethylene and absolute ethanol for 15 min, rinsed with absolute ethanol, and dried in an oven at 50 °C for 24 hours. The process was performed using DC power supply connected to an IBM computer in which current and voltage could be continuously recorded at intervals of 0.5 seconds (Fig. 1).

Said MAO process, from the first onset of micro arcing phenomenon on the titanium/titanium alloy anode to near anodic forming voltage transient with slope d*V*/d*t* ≈ 0.3, absolutely depends on specified combinations of major electrochemical parameters, especially the intrinsic nature and concentration of said electrolytes, the applied current density, and the electrolyte temperature. As typical examples, the present invention controls the anodic forming voltage transient as follows: Increases of the current density and the surface area ratio of anode to cathode resulted in an increase of the anodic forming voltage transient - consequently the anodising time, required to reach anodic forming voltage transient with slope *d*V/d*t* ≈ 0.3, becomes longer, whilst an increase of the electrolyte concentration, electrolyte temperature and agitation speed, decreases the anodic forming voltage transient - consequently, the anodising time to reach anodic forming voltage transient with slope d*V*/d*t* = 0.3, becomes shorter; decreases of the current density and the surface area ratio of anode to cathode decreases the anodic forming voltage transient - consequently, the anodising time to reach anodic forming voltage transient with slope d*V*/d*t* = 0.3, becomes shorter, whilst decreases of the electrolyte concentration, electrolyte temperature and agitation speed increase the anodic forming voltage transient - consequently, the anodising time to reach anodic forming voltage transient with slope d*V*/d*t* ≈ 0.3, becomes longer.

Therefore, any given example of specified combinations of major electrochemical parameters, especially the intrinsic nature and concentration of said electrolytes and the applied current density, are not limitative. In any cases, however, the whole anodising time did not take more than about 120 seconds. The applied DC voltage was in the range from 30 V to 500 V. The current density was in the range from 60 mA/cm² to 4000 mA/cm². The temperature was in the range from 9 °C to 100 °C. The agitation speed of the electrolytes was in the range from zero rpm to 800 rpm. The surface area ratio of the screw type of titanium anode versus the platinum counter electrodes was 3.5 %.

### Examples 1 to 10 - electrochemical parameters of the MAO process

Examples 1-5 is the MAO process in single-component calcium electrolyte systems, the electrochemical parameters of which are summarized in Table 1. Said MAO process started and ended as follows: for calcium acetate, calcium lactate, and calcium sulphate the first onset of the MAO started at 10-13 s. (in the range of approximately 210-230 V), 25-30 s. (approx. 180-200 V), 8-13 s. (approx. 120-150 V), and 10-13 s. (approx. 210-230 V), respectively. The anodizing time to maintain anodic forming voltage transient with slope d*V*/d*t* ≈ 0.3, was approximately 30-120 s., preferably 30-60 s. For electrical current densities of 60-2000 mA/cm², calcium / calcium compounds were incorporated into the anodic oxide film (maybe by a colloidal deposition mechanism). More particularly, the calcium amount of said calcium implants increased with the current density.

Examples 6-10 describe the MAO process performed in mixed calcium electrolyte systems, also summarized in Table 1. Fig. 3 shows the combinations of major parameters used to control the process: the chemical nature and concentration of calcium-containing electrolytes, applied current density, electrolyte temperature, and agitation speed. Setting the current density to 60 mA/cm² initiates the first onset of micro arcing phenomenon, which started at about 100 V (8 in Fig. 3) and ended up at 150 V (4 in Fig. 3). Correspondingly, for 300 mA/cm² it started at 130 V (7 in Fig. 3) and ended at 300 V (3 in Fig. 3); for 1000 mA/cm² it started at 260 V (6 in Fig. 3) and ended at 450 V (2 in Fig. 3); for 2000 mA/cm² it started at 330 V (5 in Fig. 3) and ended at 470 V (1 in fig 3), respectively. In all cases the process ended at d*V*/d*t* = 0.3.

**Table 1. Calcium electrolyte systems and the electrochemical parameters applied in the present invention**

| **No** | **Calcium-containing electrolytes** | **Concentration, amount, (mol/l)** | **Calcium (relative atom %)** | **Current density, (mA/cm²)** | **Voltage (V) to reach near d*V*/d*t* = 0.3** | **pH** |
|---|---|---|---|---|---|---|
| 1 | calcium hydroxide | 0.1,1.0 | 2-5 | 60, 300, 2000,4000 | 1000,150-500 | > 7.5 |
| 2 | calcium acetate | 0.025, 0.15, 1.0 | 2-10 | 60, 120, 2000 | 180,200-500 | > 7.5 |
| 3 | calcium lactate | 0.1, 0.2, 0.5, 1.0 | 2-7 | 60, 120, 2000 | 180-500 | > 7.5 |
| 4 | calcium sulphate | 0.1, 1.0 | 2-5 | 120, 1000 | 200-350 | > 7.5 |
| 5 | calcium nitrate | 0.1, 1.0 | 0.5-0.8 | 60, 120 | 30 | > 7.5 |
| 6 | calcium acetate + | 0.1 + 0.1 | 4-12 | 60, 300, 4000 | 2000, 200-500 | > 7.5 |
| | calcium hydroxide | | | | | |
| 7 | calcium acetate + EDTA | 0.1 + 0.15 | 5-20 | 60, 300, 2000 | 1000,220-500 | 7.5-10 |
| 8 | calcium acetate + EDTA malic acid | +0.1 + 0.15 + 0.1 | 8-35 | 120, 300, 2000 | 1000, 250-500 | 7.5-10 |
| 9 | calcium hydroxide + EDTA 0.1 + sodium glycerophosphate | + 0.2 0.001 | + 10-40 | 60, 300, 2000 | 1000, 220-500 | 7.5-10 |
| 10 | calcium hydroxide + EDTA 0.1 + succinic acid + glycerophosphate | + 0.2 + sodium+ 0.001 | 0.115-48 | 120, 300, 4000 | 2000, 230-500 | 7.5-10 |

### Example 11- regulation of the MAO process

The MAO process according to the present invention is designed to regulate the intensity/extension of breakdown phenomena, using the anodic voltage transient with slope *d*V/d*t* as an important parameter. The implant preparation process is characterized by an anodic voltage surge (Table 2), visible electric sparking on the anode surface, and gas evolution.

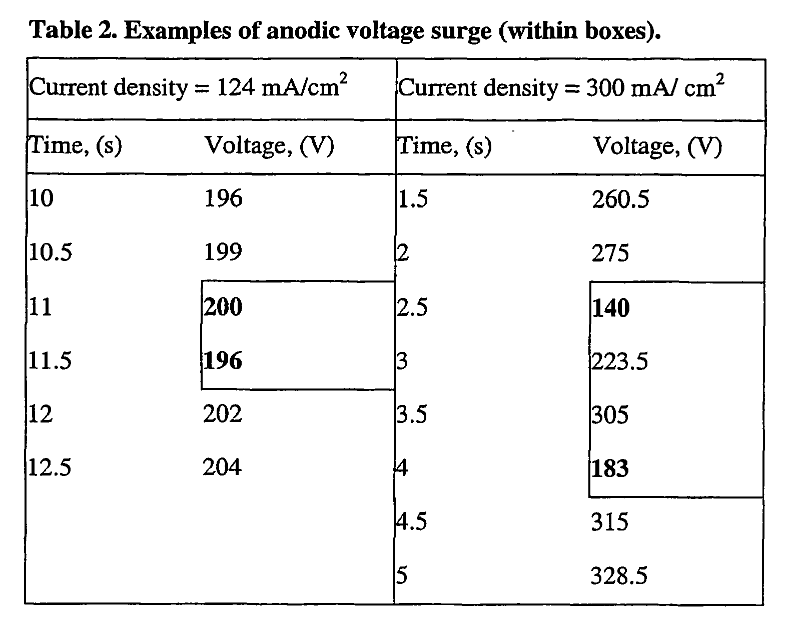

### Example 12 - surface Ca, P, and S oxide chemistry

Surface oxide chemistry of the Ca, P, and S implants was analyzed by AES and XPS measurements [Sul et al 2001b]. High-resolution XPS spectra (Fig. 7) indicated the presence of calcium titanates such as CaTiO₃ at the outermost surface for Ca implants, phosphated titanium oxides (e.g. Ti(HPO₄)₂₋ₓ, TiPO₄, etc) for P implants, and sulphated titanium oxides (e.g. TiSO₄, Ti₂(SO₄)₃, etc) for S implants, Furthermore, depth profiles by AES measurements in Fig. 8 obviously showed that Ca, P and S are incorporated throughout the titanium oxide layer by said MAO process.

### Example 13 - Transmission Electron Microscopy (TEM) cross-sectional view.

The oxide films were crosscut using an ultramicrotome and the morphological structure, as seen in the TEM cross-sectional view of Fig. 4, demonstrated a double structure. The upper porous layer (U in Fig. 4) was characterised by a lot of craters and is much thinner than the lower layer (L in Fig. 4). Furthermore, the lower barrier layer did not include any pores/craters, extension of pore channels, network of channels or connected channel branches.

### Example 14 - oxide thickness

The oxide thickness was measured with TEM and AES. The thickness of the calcium containing oxide layer consisted of the upper porous layer (U in Fig. 4) + the lower barrier layer (L in Fig. 4). Total thickness of one calcium containing oxide layer, measured by the TEM cross-sectional view of Fig. 4, was approximately 300 nm; about 100 nm of U and 200 nm of L. In another example shown in Fig. 8, the oxide thickness of Ca, P, and S implants is 1296 ± 225 nm, 1224 ± 144 nm, and 1080 ± 324 nm, respectively, measured with AES at one thread-top, one thread-valley, and in the bottom of the screw implant.

### Example 15 - crystal structure

A thin-film X-ray diffractometer (TF-XRD, Rigaku Co.), equipped with a PW3020 goniometer was employed for analyses of the titanium oxide crystal structure. Angles were scanned between 20° and 80° with a step size of 0.04°. Spectra were recorded using Cu Kα radiation (wavelength 1.54 Å). Crystal structures of said calcium implants showed amorphous, anatase, and a mixture of anatase and rutile structures (Fig. 9).

### Example 16 - surface roughness

The surface roughness was measured with confocal laser scanning profilometer (TopScan3D^{®}) as described by Wennerberg et al. The surface roughness parameter Sₐ (the height deviation from the mean plane) was 0.83 ± 0.32 µm for control implants, 1.04 ± 0.42 µm for S implants, 0.82 ± 0.29 µm for P implants, and 0.85 ± 0.32 µm for Ca implants. The corresponding S_{cx} values (the average distance between surface irregularities in the spatial direction) were 9.78 ± 1.40 µm, 12.05 ± 3.74 µm, 11.19 ± 2.33 µm, 9.83 ± 1.07 µm, respectively.

### Animal studies

Said Ca, P, and S implants, produced by said process, demonstrated much faster and stronger osseointegration as compared to conventional machine-turned implants. Ten mature (average age was 10 months) New Zealand white rabbits of both sexes were used. Each rabbit had three implants inserted in each tibia and one implant in each femur. After 6 weeks of healing time, evaluation of the bone tissue reactions to implants was performed in terms of a biomechanical test and histomorphometry. Both have been most widely accepted as excellent techniques to prove the bone to implant integration (osseointegration) in *in vivo* animal studies since the 1980's. Statistical analyses of the removal torque measurements were performed using the Tukey test for the purpose of multiple comparisons. The histomorphometric quantifications were analysed using Wilcoxon Signed Rank Test.

### Example 17 - biomechanical test (removal torque)

In removal torque techniques the applied counter-torque needed to unscrew the implant is measured. The electronic equipment used allowed torque analysis of the peak loosening torque [Johansson et al. 1998]. The Ca implants demonstrated the strongest bone response regarding removal torque test. S and P implants also showed significantly stronger bone tissue reactions in comparison to controls. As shown in Fig. 10, the multiple comparisons of the mean values between all implant groups demonstrated significant difference between test S implants vs controls (p = 0.022) and highly significant differences between test Ca implants vs controls (p = 0.0001). The mean peak values of the removal torque measurements were 16.8 Ncm (± 4.5; range 10-25 Ncm) for S implants (n =12), 15.1 Ncm (± 4.3; range 9-20 Ncm) for P implants (n = 16), 19.4 Ncm (± 3.1; range 14-24 Ncm) for Ca implants, and 12.7 Ncm (± 1.9; range 10-15 Ncm) for control implants (n = 15).

### Example 18 - histomorphometry

The histomorphometrical investigations involved (i) quantifications of the bone to metal contact (BMC) and (ii) the evaluation of tissue remodelling activity (a rough estimate of the osteoconductivity) [Johansson et al. 1998 and Sul et al 2001b]. Routinely stained undecalcified Toluidine blue sections (10-30 µm thick) were histomorphometrically analysed with a Leitz Aristoplan light microscope, which was equipped with a Leitz Microvid unit, connected to a PC, enabling the observer to perform quantifications directly in the eye-piece of the microscope, using a magnification of 10X and a zoom of 2.5X. The Ca implant demonstrated the strongest bone response regarding quantitative histomorphometry. The increase of mean BMC values revealed 272 % in Ca implants, 232 % in P implants, and 186 % in S implants when compared to the paired control groups. Said Ca, P, and S implants (Fig. 11) showed much higher osteoinductive properties than machine-turned oxide surface of non-treated implants. Histological findings (Fig. 12) clearly showed that on the modified surfaces, and most particularly the Ca implant surfaces, the bone in close contact to the implant surface was more homogeneous and much more densely mineralised while the control surface demonstrated a lot of osteoid layers and was less mineralised as seen by the staining reactions. Apposition of new bone along said implant surfaces was more diffusely spread in comparison with control implants. This indicates the superior osteoconductivity of said implants, especially Ca implants.

### Example 19 - reinforcement of mechanical properties

The mechanical strength of the oxide layer of anodised implants is an essential requirement for the usefulness and safety of clinical applications. Calcium-containing oxides made by said MAO process demonstrated excellent mechanical strength. The tensile strength of said oxide layer was shown to be 62 MPa (Table 3), which almost corresponds to a 100 % increase compared to 33MPa of SE 9901974-7 and to the range of 30 to 35 MPa of US 5478237 A.

**Table 3. Test of tensile strength for the Ca Implant**

| Sample | Surface area of titanium disc (cm²) | Strain, (MPa) |
|---|---|---|
| 1 | 4.9 | 57 |
| 2 | 4.9 | 62 |
| 3 | 4.9 | 65 |
| 4 | 4.9 | 63 |
| Mean | | 62 |
| Standard.deviation | | 3 |

Oxidised implants having thicker oxide layers than the present calcium implants (more than 5 µm, as formed below d*V*/d*t* < 0.3 in zone 3 of Fig. 2), showed a delamination of the oxide film- and oxide wear particles scattered near the implant surface (maybe due to tear-off of the thicker oxide films) internalised by the inflammatory cells such as macrophages and multinuclear giant cells (Fig. 6a). As a matter of fact, the thicker oxide layer obtained in or above zone 3 showed inferior mechanical properties such as microcracks (**e** in Fig. 2). Other oxidised implants having thinner oxide layers with thickness of 202 ± 53 nm, 608 ± 127 nm, and 805 ± 112 nm formed in zones 1 and 2 (**a** and **b** in Fig. 2) did not show "tear off" of the oxide film and no oxide wear particles could be detected.

However, the calcium-containing oxides made by said MAO process demonstrated superior mechanical strength and osseointegration properties, better than any implant tested so far, and did not exhibit a delamination or oxide particles formation (Fig. 6b).

### Example 20 - measurement of the porosity

The porosity was defined as the total area of the opening pores /the total scanned area (3 × 20 µm × 26 µm) in %. In the table below the pore characteristics of screw implants is shown. The data was obtained using an image analysis of Scanning Electron Microscopy (SEM) negatives on 3 randomly selected areas with a scanning area of 20 µm × 26 µm.

**Table 4.**

| **Sample** | **PSD¹** | **Porosity (%)²** | **Pore number³** |
|---|---|---|---|
| Group I | 1.27 µm², ≤ 8 µm | 12.7 | 156 |
| Group II | 1.53 µm², ≤ 8 µm | 24.4 | 245 |
| Group III | 2.10 µm², ≤ 8 µm | 18.7 | 139 |

| | | | |
|---|---|---|---|
| ¹Pore size distribution (PSD) was presented by opening area and by diameter. ²Porosity defined as the total area of the opening pores/the total scanned area 3 × 20 µm × 26 µm in % ³Pore number counted in the scanning area of 3 × 20 µm × 26 µm. | | | |

The invention shall not be regarded as being limited to the above embodiments and examples, but shall be interpreted within the scope of the appended claims.

### References

Adell R. et al. Int J Oral Maxillofac Impl 5, 347-359 (1990)
Kasemo B. and Lausmaa J. Swedish dental journal, Suppl. 28, 19-36 (1983).
Johansson C.B. et al. Int. J. Oral. Maxillofac. Implants 13, 315-321(1998)
Li J. et al. In: Black J, Hastings G, (eds). Handbook of Biomaterial Properties. London: Chapman & Hall; 340-354 (1998)
Hench L. et al. Chem Rev 90:33-72 (1990)
Albrektsson T.J. Oral Maxillofac Surg 56:1312-1326 (1998)
Gottlander M. et al. Clin Oral Implant Res 8: 345-351 (1997)
Sul Y.T. et al. Med. Eng. Phys, 23, 329-346 (2001 a)
Sul Y.T. et al. J Mater Sci-Mater Med;12:1025-1031 (2001b)
Hala J. and Lausmma J. Applied Osseointegration Research 1: 5-8 (2000)
Michiaki H et al. Altobia, 1, (1989)
Seishiro I. Color material, 62, (1989)
Wennerberg A et al., J Biomed Eng;14:412-418 (1992)

## Claims

1. Osteoconductive/ osteoinductive titanium/titanium alloy implant comprising:an additional element in the titanium oxide, obtained by anodic oxidation, said additional element being a single one element chosen from the group consisting of calcium, phosphor or sulphur, wherein said implant exhibits a cross-section of the osteoconductive/ osteoinductive oxide layer, which consists of a double layer structure of an upper porous layer with anodic incorporation of the additional element and a lower compact barrier layer of the titanium oxide with anodic incorporation of the additional element, wherein the upper porous layer exhibits more than about 11 % porosity and less than about 30 % porosity. and whereby the thickness of said osteoconductive/osteoinductive double layer-structured oxide containing an additional element is from 300 to 3000 nm, wherein said upper layer has a thickness of less than 1000 nm, and said lower barrier layer has a thickness ranging between 300 to 2000 nm.

2. Implant according to claim 1, wherein the upper layer has a thickness of 100-500 nm.

3. implant according to any of the preceding claims to, wherein the lower barrier layer has a thickness ranging between 600-1500 nm.

4. Implant according to any of the preceding claims, wherein the thickness of said osteoconductive/osteoinductive double layer-structured oxide containing an additional element is between 800 and 1500 nm.

5. Implant according to any of the preceding claims, wherein the lower barrier layer does not include any pores/craters or channels.

6. Implant according to any of the preceding claims, wherein the upper porous layer exhibits about 15 % porosity

7. Implant according to any of the preceding claims, wherein the crystal structure of titanium oxide is amorphous and/or amorphous and anatase and/or amorphous, anatase and rutile.

8. Implant according to any of the preceding claims, wherein the relative concentration of the additional element incorporated into the anodic oxide layer formed on titanium/titanium alloy implants increases with the thickness of the oxide layer containing an additional element, wherein the relative concentration of the additional element of the oxide layer of said implant is between 1 % and 50 %.

9. Implant according to any of the preceding claims, wherein the relative concentration.of the additional element in the oxide layer of said implant is between 1 % and 25 %.

10. A process of producing osteoconductive/osteoinductive titanium/titanium alloy implants comprising an additional compound as a titanium oxide component, wherein the additional compound is chosen from the group consisting of calcium, phosphor or sulphur, using a electrochemical oxidation method, micro arcing oxidation, comprising the steps of:
a) controlling the anodic forming voltage transient with slope (*d*V/d*t*), oxide growth constant (α, nmN), the current efficiency (nm.cm²/C), the anodic oxide forming rate (nm/sec);
b) controlling the intensity and extent of breakdown phenomenon and breakdown voltage;
c) controlling the incorporation of either calcium, phosphorous or sulphur into a double layer structure of a upper porous and a lower barrier oxide layer by colloidal deposition mechanism,
d) controlling reinforcement of the mechanical properties of said calcium-containing oxide for long term functional loading in the human body - to strictly confine the anodising time from the first onset of micro arcing phenomenon on the titanium/titanium alloy anode to near anodic forming voltage transient with slope d*V*/*d*t ≈ 0.3, during the different phases of the anodic process;
e) detecting the first onset of said micro arcing phenomenon of said process during the oxidation;
f) maintaining said process by near anodic forming voltage transient [with the slope,] *d*V/d*t* being about 0.3, as to produce an oxide layer on said titanium/titanium alloy implant exhibiting a double layer structure of an upper porous layer and a lower compact barrier layer,
g) and interrupting the oxidation process when dV/dt is between 0.2-0.4.

11. Process according any of the claims 10, wherein current density (mA/cm²) ranges from 30 to 4000 mAlcm².

12. Process according to claim 11, wherein current density (mA/cm²) ranges from 60 to 2000 mA/cm².

13. Process according to any of the claims 10-12, wherein the anodic forming voltage ranges from 30 to 500 V, preferably from 250 to 500 V.

14. Process according to any of the claims 13, wherein the anodic forming voltage ranges from 250 to 500 V.

15. Process according to any of the claims 10-14, wherein the temperature during the process ranges from 10 to 100 °C, preferably from 10 to 50 °C.

16. Process according to claim 15, wherein the temperature during the process ranges from 10 to 50 °C.

17. Process according to claims 10-16, for producing a osteoconductive/osteoinductive titanium/titanium alloy implant comprising calcium as a titanium oxide component, wherein said titanium /titanium alloy implant is electrochemically anodically oxidised in (a) at least one caicium-conteitning electrolyte and (b) ethylene diamine tetra acetic acid to yield a calcium-containing implant.

18. Process according to claim 17, wherein the calcium-containing electrolyte used in the anodising process is chosen from the group consisting of: calcium acetate, calcium carbonate, calcium chloride, calcium citrate, calcium glycerophosphate, calcium hydroxide, calcium lactate, calcium nitrate, and calcium sulphate.

19. Process according to any of the claims 17-18, wherein the anodic oxidation is performed at an alkaline pH value.

20. Process according to any of the claims 17- 19, wherein mixture used in the anodic oxidation process also includes weak acid electrolytes, chosen from the group consisting of boric acid, maleic acid, malonic acid, oxalic acid and succinic acid.

## Patentansprüche

1. Osteokonduktives/osteoinduktives Implantat aus Titan/Titanlegierung umfassend: ein zusäztliches Element in dem Titanoxid, welches durch anodische Oxidation erhalten ist, wobei das zusätzliche Element ein einziges Element ist, welches aus der Gruppe bestehend aus Calcium, Phosphor oder Sulphur ausgewählt ist, wobei das Implantat einen Querschnitt der osteokonduktiven/osteoinduktiven Oxidschicht aufweist, welcher aus einer Doppelschichtstruktur aus einer oberen, porösen Schicht mit anodischer Inkorporation des zusätzlichen Elementes und einer unteren, kompakten Sperrschicht des Titanoxids mit anodischer Inkorporation des zusätzlichen Elementes besteht, wobei die obere, poröse Schicht mehr als etwa 11 % Porösität und weniger als etwa 30 % Porösität aufweist, und wobei die Stärke des osteokonduktiven/osteoinduktiven, in Form einer Doppelschicht strukturierten Oxids, welches ein zusätzliches Element enthält, 300 bis 3000 nm beträgt, wobei die obere Schicht eine Stärke von weniger als 1000 nm und die untere Sperrschicht eine Stärke im Bereich von 300 bis 2000 nm aufweist.

2. Implantat nach Anspruch 1, wobei die obere Schicht eine Stärke von 100 bis 500 nm aufweist.

3. Implantat nach einem der vorgehenden Ansprüche, wobei die untere Sperrschicht eine Stärke im Bereich von 600 bis 1500 nm aufweist.

4. Implantat nach einem der vorgehenden Ansprüche, wobei die Stärke des osteokonduktiven/osteoinduktiven, in Form einer Doppelschicht strukturierten Oxids, welches ein zusätzliches Element enthält, zwischen 800 und 1500 nm beträgt.

5. Implantat nach einem der vorgehenden Ansprüche, wobei die untere Sperrschicht keine Poren/Krater oder Rinnen umfasst.

6. Implantat nach einem der vorgehenden Ansprüche, wobei die obere, poröse Schicht etwa 15 % Porösität aufweist.

7. Implantat nach einem der vorgehenden Ansprüche, wobei die Kristallstruktur des Titanoxids amorph und/oder amorph und Anatas und/oder amorph, Anatas und Rutil ist.

8. Implantat nach einem der vorgehenden Ansprüche, wobei die relative Konzentration des zusätzlichen Elementes, welches in der auf Implantaten aus Titan/Titanlegierung gebildeten anodischen Oxidschicht inkorporiert ist, mit der Stärke der ein zusätzliches Element enthaltenden Oxidschicht zunimmt, wobei die relative Konzentration des zusätzlichen Elementes der Oxidschicht des Implantats zwischen 1 % und 50 % beträgt.

9. Implantat nach einem der vorgehenden Ansprüche, wobei die relative Konzentration des zusätzlichen Elementes in der Oxidschicht des Implantats zwischen 1 % und 25 % beträgt.

10. Verfahren zur Herstellung osteokonduktiver/osteoinduktiver Implantate aus Titan/Titanlegierung, welche eine zusätzliche Verbindung als eine Titanoxid-Komponente umfassen, wobei die zusätzliche Verbindung aus der Gruppe bestehend aus Calcium, Phosphor oder Sulphur ausgewählt ist, wobei ein elektrochemisches Oxidationsverfahren, eine Mikro-Lichtbogenoxidation, verwendet wird, umfassend die folgenden Schritte:
a) Steuerung des die Anoden bildenden Spannungstransients mit der Neigung (dV/dt), Oxid-Wachstumskonstante (α nm/V), der Stromausbeute (nm.cm²/C), der anodischen Oxidbildungsrate (nm/sec);
b) Steuerung der Intensität und des Umfangs von Durchschlagsphänomen und Durchschlagsspannung;
c) Steuerung der Inkorporation von entweder Calcium, Phosphor oder Sulphur in eine Doppelschichtstruktur aus einer oberen, porösen Schicht und einer unteren Oxid-Sperrschicht durch einen kolloidalen Ablagerungsmechanismus,
d) Steuerung einer Verstärkung der mechanischen Eigenschaften des Calcium enthaltenden Oxids zur langfristigen, funktionellen Beladung in dem menschlichen Körper - um die Anodisierungszeit ab den ersten Beginn von Mikro-Lichtbogenphänomen auf der Titan-/Titanlegierungsanode bis ein baldiges, Anoden bildendes Spannungstransient mit der Neigung dV/dt ≈ 0,3 während der verschiedenen Phasen des Anodisierungsverfahrens streng abzugrenzen;
e) Detektieren des ersten Beginns des Mikro-Lichtbogenphänomens des Verfahrens während der Oxidation;
f) Aufrechterhalten des Verfahrens bei einem baldigen, Anoden bildenden Spannungstransient [mit der Neigung] dV/dt von etwa 0,3, um eine Oxidschicht auf dem Implantat aus Titan/Titanlegierung, welche eine Doppelschichtstruktur aus einer oberen, porösen Schicht und einer unteren, kompakten Sperrschicht aufweist, herzustellen,
g) und Unterbrechen des Oxidationsverfahrens, wenn dV/dt im Bereich von 0,2-0,4 liegt.

11. Verfahren nach Anspruch 10, wobei die Stromdichte (mA/cm²) im Bereich von 30 bis 4000 mA/cm²) liegt.

12. Verfahren nach Anspruch 11, wobei die Stromdichte (mA/cm²) im Bereich 60 bis 2000 mA/cm²) liegt.

13. Verfahren nach einem der Ansprüche 10 bis 12, wobei die die Anoden bildende Spannung im Bereich von 30 bis 500 V, vorzugsweise von 250 bis 500 V liegt.

14. Verfahren nach Anspruch 13, wobei die die Anoden bildende Spannung im Bereich von 250 bis 500 V liegt.

15. Verfahren nach einem der Ansprüche 10 bis 14, wobei die Temperatur während des Verfahrens im Bereich von 10 bis 100 °C, vorzugsweise von 10 bis 50 °C liegt.

16. Verfahren nach Anspruch 15, wobei die Temperatur während des Verfahrens im Bereich von 10 bis 50 °C liegt.

17. Verfahren nach den Ansprüchen 10 bis 16 zur Herstellung eines osteokonduktiven/osteoinduktiven Implantats aus Titan/Titanlegierung, welches Calcium als Titanoxid-Komponente umfasst, wobei das Implantat aus Titan/Titanlegierung in (a) mindestens einen Calcium enthaltenden Elektrolyten und (b) Ethylen-Diamin-Tetra-Essigsäure elektrochemisch anodisch oxidisiert wird, um ein Calcium enthaltendes Implantat zu erhalten.

18. Verfahren nach Anspruch 17, wobei der Calcium enthaltende, im Anodisierungsverfahren verwendete Elektrolyt aus der Gruppe bestehend aus: Calciumacetat, Calciumcarbonat, Calciumchlorid, Calciumcitrat, Calciumglycerophosphat, Calciumhydroxid, Calciumlactat, Calciumnitrat und Calciumsulphat ausgewählt ist.

19. Verfahren nach einem der Ansprüche 17 bis 18, wobei die anodische Oxidation bei einem alkalischen pH-Wert durchgeführt wird.

20. Verfahren nach einem der Ansprüche 17 bis 19, wobei die im anodischen Oxidationsverfahren verwendete Mischung auch schwache Säureelektrolyten umfasst, welche aus der Gruppe bestehend aus Borsäure, Maleinsäure, Malonsäure, Oxalsäure und Bernsteinsäure ausgewählt sind.

## Revendications

1. Implant du titane ostéoconducteur/ostéoinducteur/d'un alliage de titane, comprenant : un élément supplémentaire dans l'oxyde de titane, obtenu par l'oxydation anodique, ledit élément supplémentaire étant un seul élément choisi du groupe composé de calcium, de phosphore ou de soufre, où ledit implant présente une section transversale de la couche d'oxyde ostéoconducteur/ostéoinducteur qui est composée d'une structure à double couche d'une couche poreuse supérieure avec l'incorporation anodique de l'élément supplémentaire et d'une couche compacte inférieure de barrière de l'oxyde de titane avec l'incorporation anodique de l'élément supplémentaire, où la couche poreuse supérieure présente une porosité supérieure à environ 11 % et inférieure à environ 30%, où l'épaisseur dudit oxyde ostéoconducteur/ostéoinducteur avec une structure à double couche contenant un élément supplémentaire est comprise entre 300 et 3000 nm, où ladite couche supérieure présente une épaisseur inférieure à 1000 nm, et ladite couche inférieure de barrière présente une épaisseur comprise entre 300 et 2000 nm.

2. Implant selon la revendication 1, où la couche supérieure présente une épaisseur comprise entre 100 et 500 nm.

3. Implant selon l'une quelconque des revendications précédentes, où la couche inférieure de barrière présente une épaisseur comprise entre 600 et 1500 nm.

4. Implant selon l'une quelconque des revendications précédentes, où l'épaisseur dudit oxyde ostéoconducteur/ostéoinducteur avec une structure à double couche contenant un élément supplémentaire est comprise entre 800 et 1500 nm.

5. Implant selon l'une quelconque des revendications, où la couche inférieure de barrière ne comprend aucuns pores/cratères ou canaux.

6. Implant selon l'une quelconque des revendications, où la couche poreuse supérieure présente une porosité d'environ 15 %.

7. Implant selon l'une quelconque des revendications, où la structure cristalline de l'oxyde de titane est amorphe et/ou amorphe et anatase et/ou amorphe, anatase et rutile.

8. Implant selon l'une quelconque des revendications précédentes, où la concentration relative de l'élément supplémentaire incorporé dans la couche d'oxyde anodique formé sur des implants du titane/d'un alliage de titane augmente avec l'épaisseur de la couche d'oxyde contenant un élément supplémentaire, où la concentration relative de l'élément supplémentaire de la couche d'oxyde dudit implant est comprise entre 1 % et 50 %.

9. Implant selon l'une quelconque des revendications précédentes, où la concentration relative de l'élément supplémentaire dans la couche d'oxyde dudit implant est comprise entre 1 % et 25 %.

10. Procédé pour produire des implants du titane ostéoconducteur/ostéoinducteur/d'un alliage de titane, comprenant un composé supplémentaire comme composant d'oxyde de titane, où le composé supplémentaire est choisi du groupe composé de calcium, de phosphore ou de soufre, en utilisant un procédé d'oxydation électrochimique, oxydation micro-arc, comprenant les étapes de:
a) la commande de la formation anodique de transitoire de tension avec la pente (d*V*/d*t*), la constante de croissance d'oxyde (α, nm/V), le rendement actuel (nm. cm²/C), le taux de la formation de l'oxyde anodique (nm/sec);
b) la commande de l'intensité et de l'étendue du phénomène de désagrégation et de la tension de désagrégation;
c) la commande de l'incorporation soit de calcium, de phosphore soit de soufre dans une structure à double couche d'une couche d'oxyde poreuse supérieure et une couche d'oxyde inférieure de barrière par un mécanisme colloïdal de dépôt,
d) la commande du renforcement des propriétés mécaniques dudit oxyde contenant du calcium pour le chargement fonctionnel à long terme dans le corps humain - pour une limitation stricte du temps d'anodisation depuis le premier début du phénomène de micro-arc sur l'anode du titane/d'un alliage de titane à la quasi formation anodique de transitoire de tension avec la pente *dV*/*dt* ≈ 0,3 pendant les différentes phases du procédé anodique;
e) la détection du premier début dudit phénomène de micro-arc dudit procédé pendant l'oxydation;
f) maintenant ledit procédé par la quasi formation anodique de tension transitoire [avec la pente] *dV*/*dt* étant environ 0,3 afin de produire une couche d'oxide sur ledit implant du titane/d'un alliage de titane présentant une structure à double couche d'une couche poreuse supérieure et une couche compacte inférieure de barrière,
g) et l'interruption du procédé d'oxydation quand dV/dt est compris entre 0,2 et 0,4.

11. Procédé selon la revendication 10, où la densité actuelle (mA/cm²) est comprise entre 30 et 4000 mA/cm².

12. Procédé selon la revendication 11, où la densité actuelle (mA/cm²) est comprise entre 60 et 2000 mA/cm².

13. Procédé selon l'une quelconque des revendications 10 à 12, où la formation anodique de tension est comprise entre 30 et 500 V, préférablement entre 250 et 500 V.

14. Procédé selon la revendication 13, où la formation anodique de tension est comprise entre 250 et 500 V.

15. Procédé selon l'une quelconque des revendications 10 à 14, où la température pendant le procédé est comprise entre 10 et 100°C, préférablement entre 10 et 50°C.

16. Procédé selon la revendication 15, où la température pendant le procédé est comprise entre 10 et 50°C.

17. Procédé selon les revendications 10 à 16, pour produire un implant du titane ostéoconducteur/ostéoinducteur/d'un alliage de titane comprenant le calcium comme composant d'oxyde de titane, où ledit implant du titane/d'un alliage de titane est électrochimiquement et anodiquement oxydé dans (a) au moins un électrolyte contenant du calcium et (b) acide éthylènediamine tétra-acétique pour produire un implant contenant du calcium.

18. Procédé selon la revendication 17, où l'électrolyte contenant du calcium utilisé dans le procédé d'anodisation est choisi du groupe composé de: acétate de calcium, carbonate de calcium, chlorure de calcium, citrate de calcium, glycérophosphate de calcium, hydroxyde de calcium, lactate de calcium, nitrate de calcium, et sulfate de calcium.

19. Procédé selon l'une quelconque des revendications 17 à 18, où l'oxydation anodique est effectuée à une valeur pH alcaline.

20. Procédé selon l'une quelconque des revendications 17 à 19, où le mélange utilisé dans le procédé d'oxydation anodique comprend également des électrolytes d'acide faibles, choisis du groupe composé de l'acide borique, de l'acide maléique, de l'acide malonique, de l'acide oxalique et de l'acide succinique.
